# FASCICULE DE BREVET EUROPEEN

(11) **EP 2 520 564 B1**
(45) Date de publication et mention de la délivrance du brevet: **11.05.2016**
(21) Numéro de dépôt: 12179213.9
(22) Date de dépôt: 11.06.2008
(51) Int. Cl.: C07C 51/36, C07C 67/303, C07C 69/50, C07C 69/34, C07C 55/02, C07C 55/20, C07C 55/12, C07C 51/353, C07C 67/46, C07C 67/47, C07C 57/13, C07C 69/593, C07C 51/09, C07C 67/333, C07C 67/475, C07C 57/03

(54) **Procédé de synthèse de diacides à partir d'acides gras naturels**
Syntheseverfahren von Diaziden aus natürlichen Fettsäuren
Method for the synthesis of diacids from natural acids

(30) Priorité: 13.06.2007 FR 0755733
(43) Date de publication de la demande: 07.11.2012
(62) Demande divisionnaire de: 08805973.8
(73) Titulaire: Arkema France, 92700 Colombes (FR)
(72) Inventeur: Dubois, Jean-Luc, 69390 Millery (FR)

(56) Documents cités:
- US-A- 2 807 633
- US-A- 5 728 917
- STETTER, DIERICHS: "Eine neue Methode zur Darstellung langkettiger Carbonsäuren III", CHEMISCHE BERISCHTE, vol. 85, 1952, pages 1061-1067, XP002466935,
- BAKER B W ET AL: "ANODIC SYNTHESES. PART XI. SYNTHESIS OF TARICIC AND PETROSELINIC ACID", JOURNAL OF THE CHEMICAL SOCIETY, CHEMICAL SOCIETY, LONDON, GB, 1954, pages 1804-1807, XP009095205, ISSN: 0368-1769
- HELEN L NGO ET AL: "Metathesis of Unsaturated fatty Acids: Synthesis of Long-Chain Unsaturated-alpha,omega-Dicarboxylic Acids", JOURNAL OF THE AMERICAN OIL CHEMISTS' SOCIETY, JOURNAL OF THE AMERICAN OIL CHEMISTS' SOCIETY, vol. 83, no. 7, 1 juillet 2006 (2006-07-01), pages 629-634, XP002545489, ISSN: 0003-021X, DOI: 10.1007/S11746-006-1249-0
- MARTIN T. MWANGI ET AL: "Occlusion of Grubbs' Catalysts in Active Membranes of Polydimethylsiloxane: Catalysis in Water and New Functional Group Selectivities", JOURNAL OF THE AMERICAN CHEMICAL SOCIETY, vol. 128, no. 45, 1 novembre 2006 (2006-11-01), pages 14434-14435, XP55038461, ISSN: 0002-7863, DOI: 10.1021/ja0642212
- WARWEL S ET AL: "Polymers and surfactants on the basis of renewable resources", CHEMOSPHERE, PERGAMON PRESS, OXFORD, GB, vol. 43, 2001, pages 39-48, XP002262684, ISSN: 0045-6535
- CHANDRASEKHAR SOSALE ET AL: "Conformationally Restricted Criegee Intermediates: Evidence for Formation and Stereoelectronically Controlled Fragmentation", JOURNAL OF THE CHEMICAL SOCIETY, PERKIN TRANSACTIONS 2, CHEMICAL SOCIETY. LETCHWORTH, GB, vol. 10, 1 janvier 1994 (1994-01-01), pages 2141-2144, XP009095201, ISSN: 1472-779X, DOI: 10.1039/P29940002141

## Description

L'invention vise un procédé de synthèse par métathèse de diacides à longues chaînes saturées à partir d'un acide gras mono-insaturé, soit naturel, soit provenant de la transformation directe d'une huile naturelle.

Les diacides sont obtenus industriellement selon différentes méthodes, mais qui toutes présentent certains inconvénients. Un large panorama de ces méthodes est développé dans l'Encyclopédie Kirk-Othmer Vol. A8 pages 523-539.

On peut y distinguer les méthodes par dégradation telles que l'ozonolyse ou l'oxydation des acides gras végétaux.

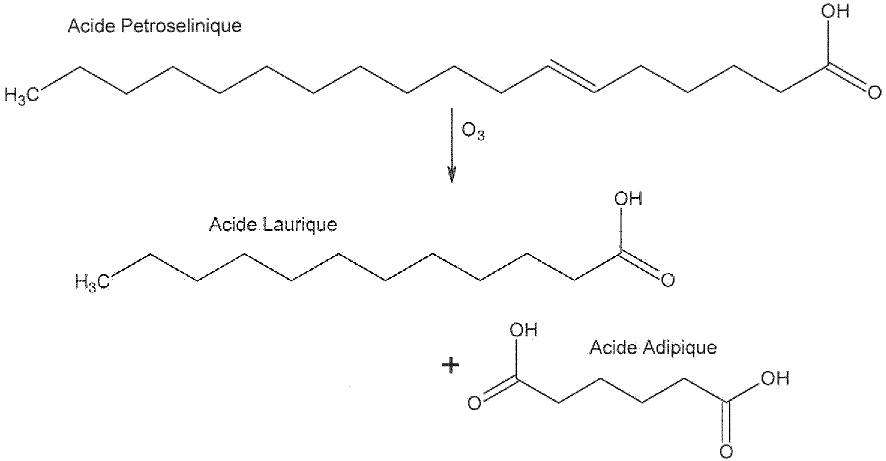

L'ozonolyse de l'acide oléique, de l'acide pétrosélinique et de l'acide érucique permet de produire respectivement les diacides à 9, 6 et 13 atomes de carbone selon le processus réactionnel ci-dessus pour l'acide pétrosélinique.

Un autre exemple est le clivage de l'acide ricinoléique par action de la soude à une température supérieure à 180°C. Cette méthode utilisée industriellement permet d'obtenir le diacide à 10 atomes de carbone.
La même méthode, telle qu'illustrée dans le schéma ci-dessous peut être appliquée à l'acide lesquérolique et conduit à la formation d'un diacide à 12 atomes de carbone. Cette méthode présente l'avantage d'utiliser des matières premières renouvelables mais est limitée essentiellement au diacide en C10, l'acide lesquérolique étant encore peu répandu, et donc cette méthode est relativement peu utilisée.

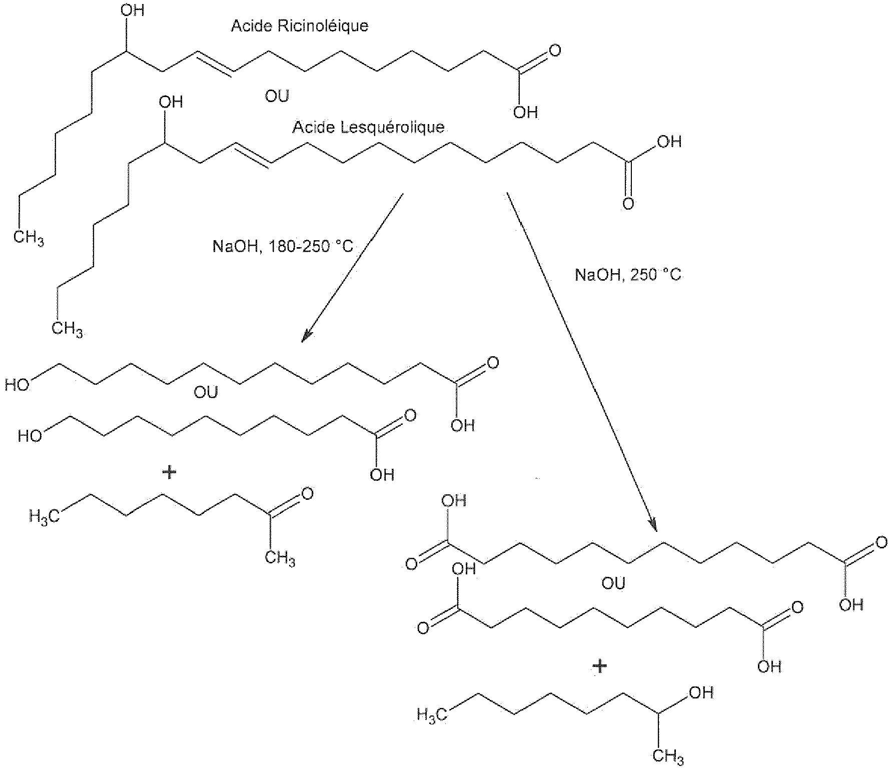

On peut citer aussi la dégradation oxydante des acides monocarboxyliques par action de N₂O₄. L'oxydation de l'acide stéarique permet d'obtenir un mélange d'acide sébacique et d'acide caprylique ; à partir d'acide palmitique on peut obtenir l'acide subérique.

Il est également possible d'obtenir des diacides à partir de molécules de plus petites tailles en utilisant des techniques variantes de la carbonylation.

Il est par ailleurs connu d'utiliser un procédé de fabrication de diacide saturé par hydrogénation d'un acide insaturé. L'article « Darstellung einiger ungesàttigter und phenylsubstituierter Carbonsäuren », de H. Stetter et W. Dierichs, dans Chemische Berischte, 1952, 85, 1061-1067, décrit un procédé d'hydrogénation du diacide insaturé HO₂C-[CH₂]₆-CH=CH-[CH₂]₆-CO₂H en diacide saturé de formule HO₂C-[CH₂]₁₄-CO₂H et l'article « Synthesis of Tariric and Petroselinic Acid », de B.W. Baker et al., dans Journal of Chemical Society, 1954, 1804-1807, décrit le procédé d'hydrogénation de l'acide dodec-cis-6-énedioïque en acide dodecanedioïque. Tout comme l'article « Conformationally Restricted Criegee Intermediates Evidence for Formation and Stereoelectronically Controlled Fragmentation », de S. Chandrasekhar et al., dans Journal of the Chemical Society, Perkin Transactions 2, 1994, 2141-2144, décrit l'hydrogénation du diester insaturé de formule CH₃OOC-(CH₂)₈-CH=CH-COOCH₃ en diester saturé.

Ces trois documents ne décrivent pas un procédé de synthèse d'une gamme complète de diacides à partir de matières premières renouvelables d'origine naturelle.

On peut citer enfin la fermentation bactérienne de paraffines, méthode bien connue et qui permet d'obtenir de nombreux diacides de longueur de chaîne variable. Cependant cette méthode ne permet pas d'obtenir des diacides de longueur supérieure à 16 atomes de carbone car les paraffines ont alors un point de fusion beaucoup trop élevé pour pouvoir être transformées. Un autre inconvénient important est que les bactéries consomment une partie des paraffines pour assurer leur croissance, conduisant à des rendements faibles et à la nécessité de purifier les produits.

Dans l'industrie des polymères, notamment pour la production de polyamides de type diacides-diamines ou de polymères techniques, il est nécessaire de disposer de toute une gamme de diacides comme matières premières, diacides qui pourront en outre être convertis en diamines de même longueur de chaîne par simple réaction chimique.

Il est donc nécessaire de trouver un type de procédé qui permet d'obtenir une gamme quasiment complète de diacides et qui, en outre, utilise des matières renouvelables d'origine naturelle.

L'objectif de l'invention est un procédé de production de toute une gamme de diacides saturés de formule générale : ROOC-(CH₂)_{X}-COOR à partir d'acides gras d'origine naturelle.

La solution proposée consiste à travailler à partir des acides gras naturels à longue chaîne mono-insaturés. On entend par acide gras naturel à longue chaîne un acide issu des milieux végétal ou animal, y compris les algues, plus généralement du règne végétal, et donc renouvelable comportant au moins 10 et de préférence au moins 14 atomes de carbone par molécule.

On peut citer à titre d'exemples de tels acides, les acides en C10, les acides obtusilique (cis-4-décénoique) et caproléique (cis-9-décénoique), les acides en C12, les acides lauroléique (cis-5-dodécénoîque) et lindérique (cis-4-dodécénoique), les acides en C14, les acides myristoléique (cis-9-tétradécénoique), physétérique (cis-5-tetradécénoique) et tsuzuique (cis-4-tetradécénoique), l'acide en C16, l'acide palmitoléique (cis-9- hexadécénoique), les acides en C18, les acides oléique (cis-9-octadécénoique), élaïdique (trans-9-octadécénoique), pétrosélinique (cis-6-octadécénoique), vaccénique (cis-11-octadécénoique) et ricinoléique (12-hydroxy--cis-9-octadécénoique), les acides en C20, les acides gadoléique (cis-9-eicosénoique), gondoique (cis-11-eicosénoique), l'acide cis-5-eicosènoique et l'acide lesquérolique (14-hydroxy-cis-11-eicosénoique), les acides en C22, les acides cétoléique (cis-11-docosénoique) et érucique (cis-13-docosénoique).

Ces divers acides sont issus des huiles végétales extraites de diverses plantes telles que le tournesol, le colza, le ricin, le lesquerella, l'olive, le soja, le palmier, la coriandre, le céleri, l'aneth, la carotte, le fenouil, le Limnanthes Alba (meadowfoam).

Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins.

L'invention vise un procédé de synthèse de diacides de formule générale ROOC-(CH₂)ₓ-COOR dans laquelle x représente un nombre entier compris entre 5 et 24, et R est H, à partir d'acides gras naturels à longue chaîne mono-insaturés comportant au moins 10 atomes de carbone adjacents par molécule, de formule CH₃-(CH₂)ₙ-CHR₁-CH₂-CH=CH-(CH₂)ₚ-COOR, dans laquelle R représente H, R₁ est soit H, soit OH, et n et p, identiques ou différents, sont des indices compris entre 3 et 11 consistant à transformer dans une première étape, ledit acide gras naturel, soit par pyrolyse soit par éthénolyse (métathèse croisée avec l'éthylène), en un acide gras ω-mono-insaturé de formule générale CH₂=CH-(CH₂)ₘ-COOR dans laquelle m est égal à p ou p+1 selon la nature de l'acide gras traité et la transformation utilisée, éthénolyse ou pyrolyse, puis dans une deuxième étape à soumettre le produit ainsi obtenu à une réaction de métathèse, soit homométathèse pour obtenir un composé de formule ROOC-(CH₂)ₘ-CH=CH-(CH₂)ₘ-COOR, soit métathèse croisée avec un composé de formule R₂OOC-(CH₂)ᵣ-CH=CH-R₃ dans laquelle R₂ est H, r est soit 0, soit 1 soit 2 et R₃ est H, CH₃ ou COOR₂ formant dans ce dernier cas une molécule non cyclique, pour obtenir un composé insaturé de formule ROOC-(CH₂)ₘ-CH=CH-(CH₂)ᵣ-COOR₂ , puis dans une troisième étape enfin à transformer par hydrogénation de la double liaison le composé insaturé en composé saturé.

L'acide gras mono-insaturé naturel de formule générale CH₃-(CH₂)ₙ-CHOH-CH₂-CH=CH-(CH₂)ₚ₋-COOR peut être soumis à une réaction de pyrolyse.

L'acide de formule CH₂=CH-(CH₂)ₚ₊₁-COOR issu de la première étape peut être soumis à une homométathèse dont le produit ROOC-(CH₂)ₚ₊₁-CH=CH-(CH₂)ₚ₊₁-COOR est hydrogéné.

L'acide de formule CH₂=CH-(CH₂)ₚ₊₁-COOR issu de la première étape peut être soumis à une métathèse croisée dont le produit obtenu est hydrogéné.

L'acide gras mono-insaturé naturel de formule générale CH₃-(CH₂)ₙ-CHOH-CH₂-CH=CH-(CH₂)ₚ₋-COOR peut être soumis à une réaction d'éthénolyse.

L'acide de formule CH₂=CH-(CH₂)ₚ-COOR issu de la première étape peut être soumis à une homométathèse dont le produit ROOC-(CH₂)ₚ-CH=CH-(CH₂)ₚ-COOR est hydrogéné.

L'acide de formule CH₂=CH-(CH₂)ₚ-COOR issu de la première étape peut être soumis à une métathèse croisée dont le produit obtenu est hydrogéné.

La métathèse croisée est faite avec l'acide acrylique quand R₂=H, x=0 et R₃=H. Dans le cas où x=1, R₂=H et R₃=CH₃, le composé est HOOC-CH₂-CH=CH-CH₃ et est obtenu par exemple par hydroxycarbonylation du butadiène. Dans ce cas, au cours de la métathèse croisée, du propylène est produit et est éliminé du milieu réactionnel.

De préférence, lorsque R₃ est COOR₂, R₂OOC-(CH₂)ᵣ-CH=CH-R₃ est une molécule symétrique avec r=0. Lorsque R₃ est CH₃, R₂OOC-(CH₂)ᵣ-CH=CH-R₃, réagit avec un acide gras par métathèse croisée, la réaction conduit à un diacide et un acide gras plus court, mais aussi à du propylène. Le propylène est éliminé au fur et à mesure du milieu réactionnel ce qui déplace la réaction vers les produits souhaités.

Lorsque R₂OOC-(CH₂)ᵣCH=CH-COOR₂ forme une molécule cyclique comme l'anhydride maléique, alors la métathèse croisée conduit à un acide gras insaturé contenant aussi une fonction anhydride. Le diacide et l'acide gras peuvent être libérés par hydrolyse.

Dans le procédé de l'invention, on peut traiter l'acide gras soit sous sa forme acide soit sous sa forme ester. Le passage d'une forme à l'autre s'effectue par méthanolyse, estérification ou hydrolyse.

Dans le procédé de l'invention on utilise des acides ou esters gras d'origine naturelle, c'est-à-dire contenus dans des huiles ou des graisses d'extraction. Ces dernières sont en fait constituées à côté de l'ester ou de l'acide entrant dans la réaction d'un mélange d'esters ou acides de formules voisines. A titre d'exemples l'huile de palme contient outre l'acide oléique de l'acide linoléique; l'huile de ricin contient outre l'acide ricinoléique à la fois de l'acide oléique et l'acide linoléique, l'huile de colza contient outre l'acide oléique à la fois de l'acide linoléique, de l'acide linolénique et de l'acide gadoléique. La présence de ces acides di-insaturés ou poly-insaturés n'a pas de conséquence importante sur le déroulement du procédé dans la mesure où lors de la première étape en cas d'éthénolyse, l'acide linoléique formera aussi l'acide gras ω-mono-insaturé de formule générale CH₂=CH-(CH₂)ₘ-COOR avec des quantités mineures de diènes courts et d'α-oléfines. Dans le cas de l'acide ricinoléique la réaction de pyrolyse ne transformera pas ces acides voisins.

Des exemples de synthèse de diacides sont donnés ci après. Tous les mécanismes détaillés ci-dessous illustrent, pour faciliter l'exposé, la forme acide. Cependant, la métathèse est aussi efficace avec un ester et même souvent plus efficace, le milieu étant généralement plus anhydre. De la même manière, les schémas illustrent des réactions avec l'isomère cis des acides; les mécanismes sont aussi bien applicables aux isomères trans.

Le diacide en C6 peut être obtenu à partir des acides obtusilique (cis-4-décénoique), lindérique (cis-4-dodécénoique) et tsuzuique (cis-4-tetradécénoique) en réalisant une éthénolyse en première étape, suivie d'une métathèse croisée avec l'acide acrylique puis hydrogénation.

Le diacide en C7 peut être obtenu à partir des acides lauroléique (cis-5-dodécénoique) et physétérique (cis-5-tetradécénoique) par une éthénolyse en première étape, suivie d'une métathèse croisée avec l'acide acrylique puis hydrogénation.

Le diacide en C8 peut être obtenu à partir des acides obtusilique (cis-4-décénoique), lindérique (cis-4-dodécénoique) et tsuzuique (cis-4-tetradécénoique) en réalisant une éthénolyse en première étape, suivie d'une homométathèse ou à partir de l'acide pétrosélinique par éthénolyse en première étape, suivie d'une métathèse croisée avec l'acide acrylique et dans les deux cas achevée par une hydrogénation.

Le diacide en C10 peut être obtenu à partir des acides lauroléique (cis-5-dodécénoique) et physétérique (cis-5-tetradécénoique) par une éthénolyse en première étape, suivie d'une homométathèse complétée par l'hydrogénation.

Le diacide en C11 peut être obtenu à partir des acides oléique (cis-9-octadécénoique), élaidique (trans-9-octadécénoique), acide gadoléique (cis-9-eicosénoique) et les acides myristoléique (cis-9-tétradécénoique) avec une éthénolyse en première étape suivie d'une métathèse croisée avec l'acide acrylique et dans chaque cas achevée par une hydrogénation. Dans le cas de l'acide oléique le processus réactionnel suivant sera mis en oeuvre :
1)

   CH₃-(CH₂)₇-CH=CH-(CH₂)₇-COOH + CH₂=CH₂ ⇔ CH₂=CH-(CH₂)₇-COOH + CH₂=CH-(CH₂)₇-CH₃
2)

   CH₂=CH-(CH₂)₇-COOH + HOOC-CH=CH₂ ⇔ HOOC-CH=CH-(CH₂)₇-COOH + CH₂=CH₂
3)

   HOOC-CH=CH-(CH₂)₇-COOH + H₂ → HOOC-(CH₂)₉-COOH

Le mécanisme réactionnel de cette réaction dans ses différentes variantes est illustrée par le schéma 1 ci-dessous

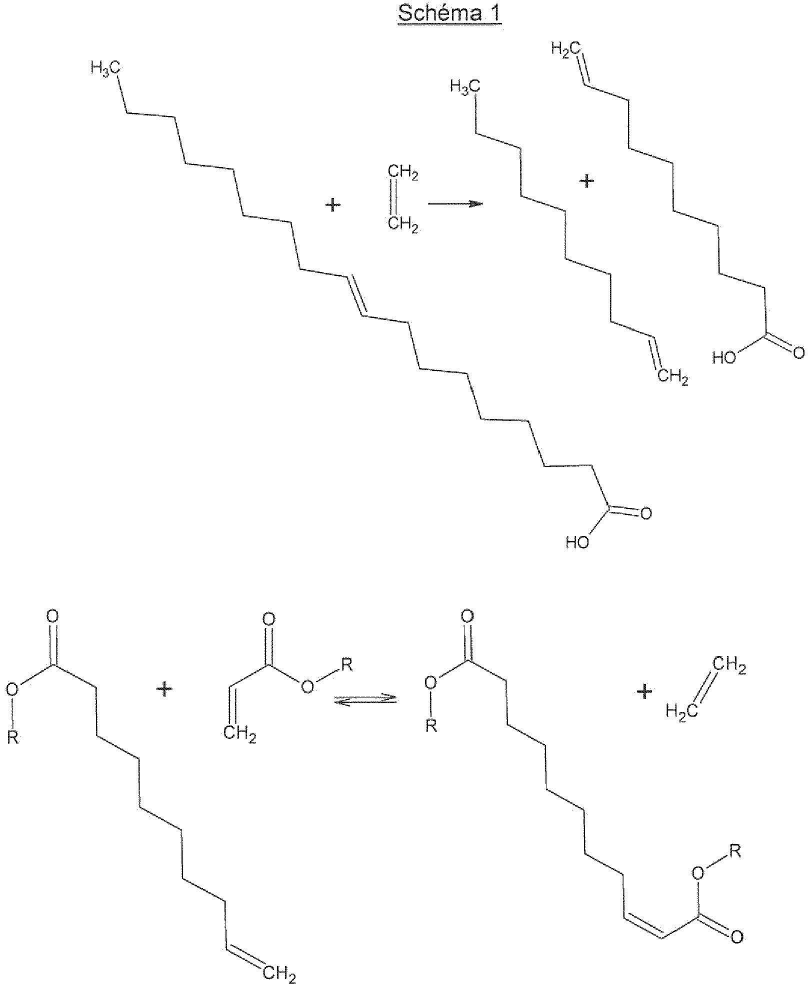

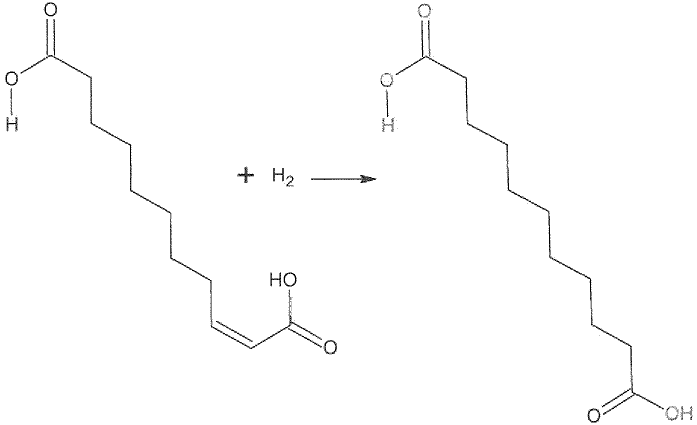

Le diacide en C12 peut être obtenu à partir des acides pétrosélinique et ricinoléique selon deux mécanismes réactionnels différents. L'acide pétrosélinique (cis-6-octadécénoique) est transformé par une éthénolyse en première étape, suivie d'une homométathèse, complétée par l'hydrogénation. L'acide ricinoléique (12-hydroxy--cis-9-octadécénoique) est quant à lui soumis à une pyrolyse permettant la synthèse de l'acide ω-undécènoique qui est soumis à une métathèse croisée avec l'acide acrylique donnant l'acide 11-dodécènedioique transformé par hydrogénation en acide dodécanedioique.

Le mécanisme réactionnel de cette réaction avec l'acide pétrosélinique (schéma 2) est le suivant.
1)

   CH₃-(CH₂)₁₀-CH=CH-(CH₂)₄-COOH + CH₂=CH₂ ⇔ CH₂=CH-(CH₂)₄-COOH + CH₂=CH-(CH₂)₁₀-CH₃
2)

   2 CH₂=CH-(CH₂)₄-COOH ⇔ HOOC-(CH₂)₄-CH=CH-(CH₂)₄-COOH + CH₂=CH₂
3)

   HOOC-(CH₂)₄-CH=CH-(CH₂)₄-COOH + H₂ → HOOC-(CH₂)₁₀-COOH

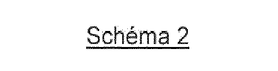

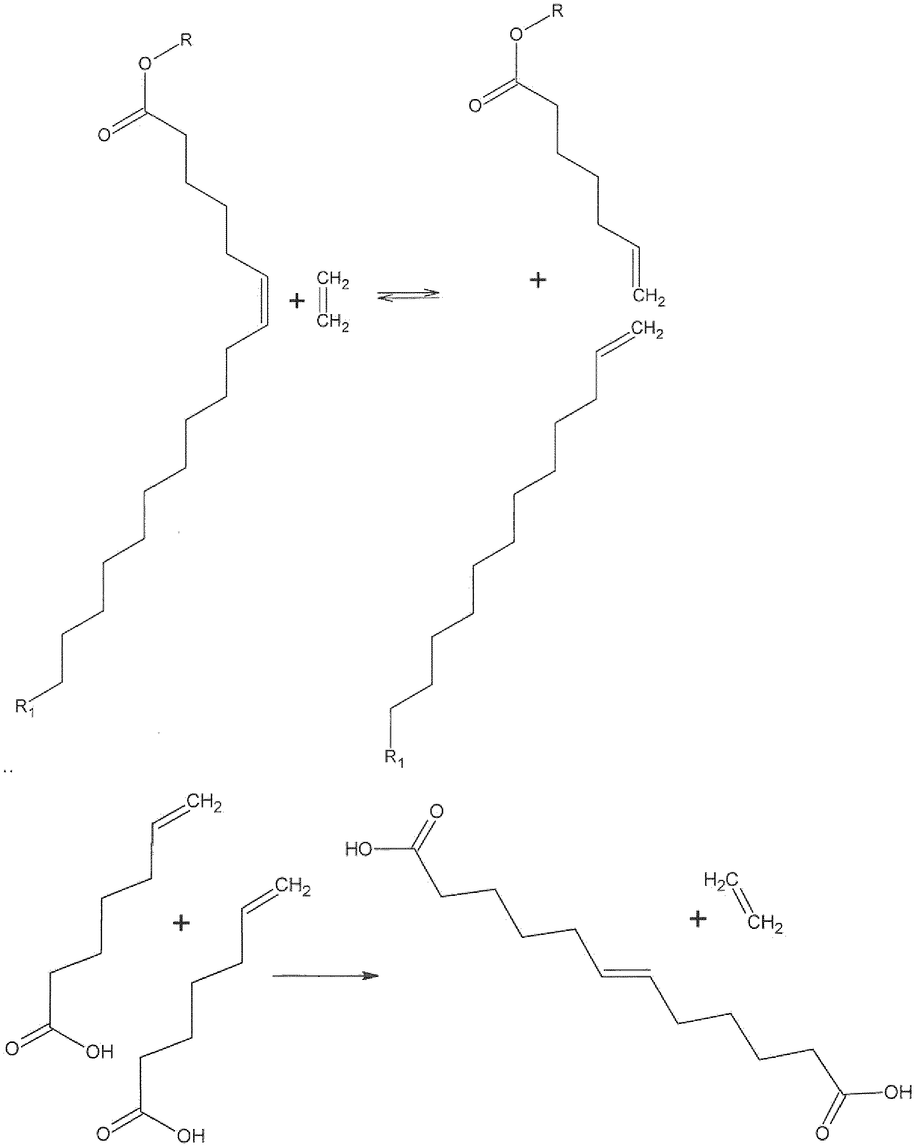

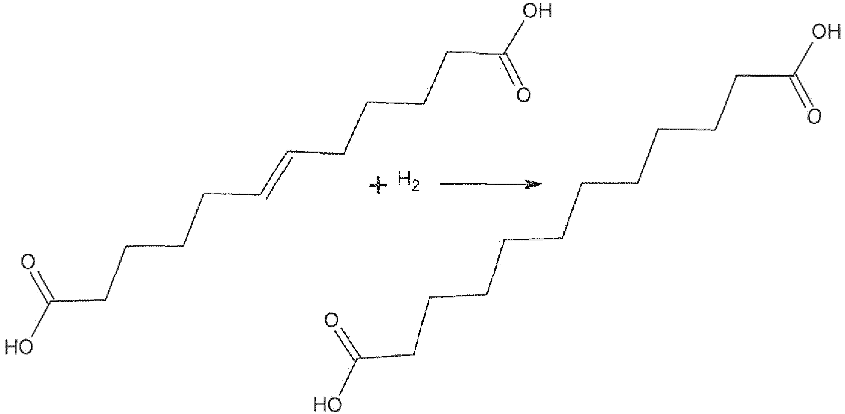

Le processus réactionnel avec l'acide ricinoléique est le suivant :
1)

   CH₃-(CH₂)₅-CHOH-CH₂-CH=CH-(CH₂)₇-COOH (Δ) → CH₃-(CH₂)₅-CHO + CH₂=CH-(CH₂)₈-COOH
2)

   CH₂=CH-(CH₂)₈COOH + HOOC-CH=CH₂ ⇔ HOOC-CH=CH-(CH₂)₈-COOH + CH₂=CH₂
3)

   HOOC-CH=CH-(CH₂)₈-COOH + H₂ → HOOC-(CH₂)₁₀-COOH.

Le diacide en C12 peut encore être obtenu par éthénolyse de l'acide oléique pour donner l'acide insaturé CH₂=CH-(CH₂)₇-COOH suivie d'une métathèse croisée avec l'acide CH₃-CH=CH-CH₂-COOH et enfin d'une hydrogénation.

Le diacide en C13 peut être obtenu à partir des acides vaccénique (cis-11-octadécénoique), gondoique (cis-11-eicosénoique) et cétoléique (cis-11-docosénoique) avec une éthénolyse en première étape suivie d'une métathèse croisée avec l'acide acrylique et dans chaque cas achevée par une hydrogénation.

Le diacide en C14 peut être obtenu à partir de l'acide lesquérolique, avec une pyrolyse de l'acide gras hydroxylé pour former l'acide de formule CH₂=CH-(CH₂)₁₀-COOCH₃ suivie d'une métathèse croisée avec l'acide acrylique et enfin d'une hydrogénation. Il peut encore être obtenu par éthénolyse de l'acide vaccénique pour donner l'acide insaturé CH₂=CH-(CH₂)₉-COOH suivie d'une métathèse croisée avec l'acide CH₃-CH=CH-CH₂-COOH et enfin d'une hydrogénation.

Le diacide en C15 peut être obtenu à partir de l'acide érucique, avec une éthénolyse en première étape suivie d'une métathèse croisée avec l'acide acrylique et achevée par une hydrogénation.

Le diacide en C16 peut être obtenu à partir de l'acide nervonique, avec une éthénolyse en première étape suivie d'une métathèse croisée avec l'acide acrylique et achevée par une hydrogénation.

Il est parfaitement possible, en mettant en oeuvre le procédé de l'invention, de fabriquer, si besoin est, des diacides supérieurs en C18, C20, C22 ou C26 par exemple.

L'invention concerne également un procédé de synthèse du diacide de formule ROOC-(CH₂)₈-COOR à partir de l'acide 5-lauroléique ou 5-physétérique avec en première étape une éthénolyse dudit acide pour produire l'acide de formule CH₂=CH-(CH₂)₃-COOR, suivie d'une homométathèse complétée par l'hydrogénation.

Les réactions de métathèse sont connues depuis longtemps même si leurs applications industrielles sont relativement limitées. On peut se référer à propos de leur utilisation dans la transformation des acides (esters) gras, à l'article de J.C. Mol « Catalytic metathesis of unsaturated fatty acid esters and oil » paru dans Topics in Catalysis Vol. 27, Nos. 1-4, February 2004 (Plénum Publishing Corporation).

La catalyse de la réaction de métathèse a fait l'objet de très nombreux travaux et le développement de systèmes catalytiques sophistiqués. On peut citer par exemple les complexes au tungstène développés par Schrock et al J. Am. Chem. Soc. 108 (1986) 2771, ou Basset et al. Angew. Chem., Ed. Engl. 31 (1992) 628. Plus récemment, sont apparus les catalyseurs dits de Grubbs (Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hétérogènes à base de métaux tels que rhénium, molybdène et tungstène déposés sur alumine ou silice. Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est d'augmenter la sélectivité de la réaction vis-à-vis des réactions parasites telles que les « homo-métathèses » entre les réactifs mis en présence. Ils portent non seulement sur la structure des catalyseurs mais également sur l'incidence du milieu réactionnel et les additifs pouvant être introduits.

Dans le procédé de l'invention, tout catalyseur de métathèse actif et sélectif pourra être utilisé. On utilisera cependant de préférence des catalyseurs à base de ruthénium et de rhénium.

La réaction d'éthénolyse (métathèse) de la première étape est conduite à une température comprise entre 20 et 100°C à une pression de 1 à 30 bars en présence d'un catalyseur de métathèse classique. Le temps de réaction est choisi en fonction des réactifs mis en oeuvre et pour atteindre au plus près l'équilibre de la réaction. La réaction est effectuée sous une pression d'éthylène.

La réaction de pyrolyse de première étape est réalisée à une température généralement comprise entre 400 et 600°C.

La réaction d'homométathèse de la deuxième étape est réalisée à une température généralement comprise entre 20 et 200°C en présence d'un catalyseur de métathèse classique.

La réaction de métathèse croisée de la deuxième étape est réalisée à une température généralement comprise entre 20 et 200°C en présence d'un catalyseur à base de ruthénium.

La réaction d'hydrogénation de troisième étape est réalisée à une température généralement comprise entre 20 et 300°C sous pression d'hydrogène, en présence d'un catalyseur contenant par exemple du nickel, du cobalt, du platine ou du palladium...
Le procédé de l'invention est illustré par les exemples suivants.

### Exemple 1 (non conforme à l'invention)

Cet exemple illustre la synthèse du diacide en C11 à partir de l'acide oléique. Dans une première étape on conduit l'éthénolyse de l'acide oléique à 30°C en présence d'un catalyseur à base de tungstène pour obtenir l'acide 9-décénoique CH₂=CH-(CH₂)₇-COOH. Pour la deuxième étape, on utilise le catalyseur complexe bispyridine ruthénium (8) décrit dans la publication de Chen-Xi Bai et al., Tetrahedron Letters, 46 (2005) 7225-7228 pour réaliser la métathèse croisée de l'acide 9-décénoique avec l'acrylate de méthyle. La réaction est effectuée dans CH₂Cl₂, à une concentration de 0,1 M d'acide 9-décénoique et de 0,2 M d'acrylate de méthyle, à une température de 50°C, et pendant 12 heures. Les rendements sont déterminés par analyse chromatographique. Dans le cas présent, on utilise 2 équivalents d'acrylate de méthyle par rapport à l'acide, et avec une concentration en catalyseur de 0,5 % mole. Le rendement en produit CH₃-OOC-CH=CH-(CH₂)₇-COOH est de 50 % molaire. Ce produit pourra être hydrogéné selon un processus classique avec un rendement de 100 %.

### Exemple 2 (non conforme à l'invention)

Cet exemple illustre la synthèse du diacide en C20 à partir de l'acide ricinoléique. Au cours de la première étape le ricinoléate de méthyle est soumis à une pyrolyse à une température de 550°C pour former le 10-undécénoate de méthyle qui est passé en forme acide par hydrolyse. Dans la deuxième étape d'homométathèse, on utilise le catalyseur complexe ruthénium (3) décrit dans la publication de Stefan Randl et al, Synlett (2001) 10, 430 qui est très stable et ne se décompose pas lorsqu'il est exposé à l'air ou à l'eau. La réaction d'homométathèse est effectuée dans CH₂Cl₂, à une concentration de 0,15 M d'acide 10-undécénoique, à une température de 30°C, et pendant 2 heures avec une concentration en catalyseur de 0,5 % mole. Les rendements sont déterminés par analyse chromatographique. Le rendement en diacide HOOC-(CH₂)₈-CH=CH-(CH₂)₈-COOH est de 67 % molaire. Ce produit pourra être hydrogéné selon un processus classique avec un rendement de 100 %.

### Exemple 3 (non conforme à l'invention)

Cet exemple illustre la synthèse du diacide en C12 à partir de l'acide ricinoléique. La première étape est identique à celle de l'exemple 2 à la réserve près que c'est l'ester méthylique de l'acide 10-undécénoique CH₂=CH-(CH₂)₈-COOCH₃ qui est adressé en deuxième étape. Cette deuxième étape est une métathèse croisée avec l'acrylate de méthyle. On utilise pour cette réaction le catalyseur complexe bispyridine ruthénium (8) décrit dans la publication de Chen-Xi Bai et al., Org. Biomol. Chem., (2005), 3, 4139-4142. La réaction est effectuée dans CH₂Cl₂, à une concentration de 0,05 M d'ester méthylique de l'acide 10-undécénoique et 0,1 M d'acrylate de méthyle à une température de 30°C, et pendant 12 heures, en présence du catalyseur à une concentration de 1 % mole par rapport à l'ester méthylique de l'acide 10-undécénoique. Les rendements sont déterminés par analyse chromatographique. Le rendement en diester CH₃-OOC-CH=CH-(CH₂)₈-COOCH₃ est de 70 % molaire. Ce produit, sous sa forme ester ou acide, pourra être hydrogéné selon un processus classique avec un rendement de 100 %.

Cet exemple illustre ainsi un procédé de synthèse du diester de formule CH₃OOC-(CH₂)₁₀ -COOCH₃ à partir de l'ester méthylique de l'acide ricinoléique soumis en première étape à une pyrolyse pour former l'ester de formule CH₂=CH-(CH₂)₈-COOCH₃ qui est ensuite soumis à une métathèse croisée avec l'acrylate de méthyle formant le diester de formule CH₃OOC-CH=CH-(CH₂)₈-COOCH₃ qui est ensuite hydrogéné.

### Exemple 4 (non conforme à l'invention)

Les catalyseurs de métathèse A et B, ont été obtenus auprès de Sigma Aldrich, référence catalogue 569747 et 569755 respectivement. Ces catalyseurs sont aussi connus comme les catalyseurs Grubbs 2nd génération et Hoveyda-Grubbs 2nd generation.
Catalyseur A : benzylidene[1,3-bis(2,4,6-trimethylphenyl)-2-imidazolidinylidene] dichloro(tricyclohexyphosphine)ruthenium.
Catalyseur B : (1,3-bis(2,4,6-trimethylphenyl)-2-imidazolidinylidenejdichloro(o-isopropoxy-phenylmethylene)ruthenium.

L'acide undécylénique est produit par Arkema par hydrolyse de l'ester méthylique de l'acide undécylénique, lui même obtenu par craquage de l'ester méthylique de l'acide ricinoléique. Ce dernier est obtenu par transestérification de l'huile de ricin par du méthanol en catalyse basique. Ces produits sont produits dans l'usine Arkema de Marseille Saint-Menet.

Dans les expérimentations, 2.5 g d'ester d'acide gras (acide undécylénique) et/ou un excès d'acrylate de méthyle sont utilisés. Le tétradécane est utilisé comme étalon interne. Le mélange réactionnel est agité à 50 °C et dégazé par de l'argon. Le catalyseur est ajouté à la solution, sans ajout de solvant. Les échantillons de produits de la réaction sont analysés par chromatographie.

Les exemples N et M ci-dessous illustrent le cas de l'homométathèse de l'undécylénate de méthyle, l'exemple O illustre le cas de la métathèse croisée de l'undécylénate de méthyle et de l'acrylate de méthyle.

| Exemple | Catalyseur (mole %) | Ratio molaire Acrylate de méthyle/und écylénate de méthyle | Conversion | Rendement d'homométa thèse | Rendement de métathèse croisée | Durée de réaction |
|---|---|---|---|---|---|---|
| | | | % mole | % mole | % mole | Min |
| | | | | | | |
| N | A (1) | 0 | 98 | 100 | 0 | 30 |
| M | B (1) | 0 | 95 | 100 | 0 | 30 |
| 0 | B(0.1) | 10 | 99 | 0 | 99 | 30 |

## Revendications

1. Procédé de synthèse de diacides de formule générale ROOC-(CH₂)ₓ-COOR dans laquelle x représente un nombre entier compris entre 5 et 24 et R est H, à partir d'acides gras naturels à longue chaîne mono-insaturés comportant au moins 10 atomes de carbone adjacents par molécule, de formule CH₃-(CH₂)ₙ-CHR₁-CH₂-CH=CH-(CH₂)ₚ₋COOR, dans laquelle R représente H, R₁ est, soit H, soit OH et n et p, identiques ou différents, sont des indices compris entre 3 et 11, consistant à transformer dans une première étape, ledit acide gras naturel, soit par pyrolyse, soit par éthénolyse, en un acide gras ω-mono-insaturé de formule générale CH₂=CH-(CH₂)ₘ-COOR dans laquelle m est égal à p ou p+1 selon la nature de l'acide gras traité et la transformation utilisée, puis dans une deuxième étape à soumettre le produit ainsi obtenu à une réaction de métathèse, soit homométathèse pour obtenir un composé de formule ROOC-(CH₂)ₘ-CH=CH-(CH₂)ₘ-COOR, soit métathèse croisée avec un composé de formule R₂OOC-(CH₂)ᵣ-CH=CH-R₃ dans laquelle R₂ est H, r est soit 0, soit 1 soit 2 et R₃ est H, CH₃ ou COOR₂ formant, dans ce dernier cas, une molécule non cyclique, pour obtenir un composé insaturé de formule ROOC-(CH₂)ₘ-CH=CH-(CH₂)ᵣ-COOR₂, puis dans une troisième étape enfin à transformer par hydrogénation de la double liaison le composé insaturé en composé saturé.

2. Procédé selon la revendication 1 **caractérisé en ce que** l'acide mono-insaturé naturel de formule générale CH₃-(CH₂)ₙ-CHOH-CH₂-CH=CH-(CH₂)ₚ₋-COOR est soumis à une réaction de pyrolyse.

3. Procédé selon la revendication 2 **caractérisé en ce que** l'acide de formule CH₂=CH-(CH₂)ₚ₊₁-COOR issu de la première étape est soumis à une homométathèse dont le produit ROOC-(CH₂)ₚ₋₁-CH=CH-(CH₂)ₚ₊₁-COOR est hydrogéné.

4. Procédé selon la revendication 2 **caractérisé en ce que** l'acide de formule CH₂=CH-(CH₂)ₚ₊₁-COOR issu de la première étape est soumis à une métathèse croisée dont le produit obtenu est hydrogéné.

5. Procédé selon la revendication 1 **caractérisé en ce que** l'acide gras mono-insaturé naturel de formule générale CH₃-(CH₂)ₙ-CHOH-CH₂-CH=CH-(CH₂)ₚ₋-COOR est soumis à une réaction d'éthénolyse.

6. Procédé selon la revendication 5 **caractérisé en ce que** l'acide de formule CH₂=CH-(CH₂)ₚ-COOR issu de la première étape est soumis à une homométathèse dont le produit ROOC-(CH₂)ₚ-CH=CH-(CH₂)ₚ-COOR est hydrogéné.

7. Procédé selon la revendication 5 **caractérisé en ce que** l'acide de formule CH₂=CH-(CH₂)ₚ-COOR issu de la première étape est soumis à une métathèse croisée dont le produit obtenu est hydrogéné.

8. Procédé selon la revendication 1 de synthèse du diacide de formule ROOC-(CH₂)₁₂-COOR à partir de l'acide vaccénique avec en première étape une éthénolyse de cet acide pour donner l'acide insaturé CH₂=CH-(CH₂)₉-COOR suivie en deuxième étape d'une métathèse croisée avec l'acide CH₃-CH=CH-CH₂-COOR et enfin d'une hydrogénation du produit de deuxième étape.

9. Procédé selon la revendication 1 de synthèse du diacide de formule ROOC-(CH₂)₈-COOR à partir de l'acide 5-lauroléique ou 5-physétérique avec en première étape une éthénolyse dudit acide pour produire l'acide de formule CH₂=CH-(CH₂)₃-COOR, suivie d'une homométathèse complétée par l'hydrogénation.

## Patentansprüche

1. Verfahren zur Synthese von Disäuren der allgemeinen Formel: ROOC-(CH₂)ₓ-COOR, worin x für eine ganze Zahl zwischen 5 und 24 steht und R für H steht, aus natürlichen langkettigen einfach ungesättigten Fettsäuren mit mindestens 10 benachbarten Kohlenstoffatomen pro Molekül der Formel CH₃-(CH₂)ₙ-CHR₁-CH₂-CH=CH-(CH₂)ₚ-COOR, worin R für H steht, R₁ entweder für H oder für OH steht und n und p gleich oder verschieden sind und für Indices zwischen 3 und 11 stehen, bei dem man in einem ersten Schritt die natürliche Fettsäure entweder durch Pyrolyse oder durch Ethenolyse in eine einfach ω-ungesättigte Fettsäure der allgemeinen Formel CH₂=CH-(CH₂)ₘ-COOR, worin m je nach der Beschaffenheit der behandelten Fettsäure und der verwendeten Umwandlung gleich p oder p+1 ist, umwandelt und dann in einem zweiten Schritt das so erhaltene Produkt einer Metathesereaktion, entweder einer Homomethathese, wobei man eine Verbindung der Formel ROOC-(CH₂)ₘ-CH=CH-(CH₂)ₘ-COOR erhält, oder einer Kreuzmetathese mit einer Verbindung der Formel R₂OOC-(CH₂)ᵣ-CH=CH-R₃, worin R₂ für H steht, r entweder für 0 oder für 1 oder für 2 steht und R₃ für H, CH₃ oder COOR₂ steht, wobei sich in diesem letzteren Fall ein acyclisches Molekül ergibt, wobei man eine ungesättigte Verbindung der Formel ROOC-(CH₂)ₘ-CH=CH-(CH₂)ᵣ-COOR₂ erhält, unterwirft und dann in einem dritten Schritt schließlich die ungesättigte Verbindung durch Hydrierung der Doppelbindung in eine gesättigte Verbindung umwandelt.

2. Verfahren nach Anspruch 1, **dadurch gekennzeichnet, dass** man die natürliche einfach ungesättigte Säure der allgemeinen Formel CH₃-(CH₂)ₙ-CHOH-CH₂-CH=CH-(CH₂)ₚ-COOR einer Pyrolysereaktion unterwirft.

3. Verfahren nach Anspruch 2, **dadurch gekennzeichnet, dass** man die Säure der Formel CH₂=CH-(CH₂)ₚ₊₁-COOR aus dem ersten Schritt einer Homometathese unterwirft und deren Produkt ROOC-(CH₂)ₚ₊₁-CH=CH-(CH₂)ₚ₊₁-COOR hydriert.

4. Verfahren nach Anspruch 2, **dadurch gekennzeichnet, dass** man die Säure der Formel CH₂=CH-(CH₂)ₚ₊₁-COOR aus dem ersten Schritt einer Kreuzmetathese unterwirft und deren Produkt hydriert.

5. Verfahren nach Anspruch 1, **dadurch gekennzeichnet, dass** man die natürliche einfach ungesättigte Fettsäure der allgemeinen Formel CH₃-(CH₂)ₙ-CHOH-CH₂-CH=CH-(CH₂)ₚ-COOR einer Ethenolysereaktion unterwirft.

6. Verfahren nach Anspruch 5, **dadurch gekennzeichnet, dass** man die Säure der Formel CH₂=CH-(CH₂)ₚ-COOR aus dem ersten Schritt einer Homometathese unterwirft und deren Produkt ROOC-(CH₂)ₚ-CH=CH-(CH₂)ₚ-COOR hydriert.

7. Verfahren nach Anspruch 5, **dadurch gekennzeichnet, dass** man die Säure der Formel CH₂=CH-(CH₂)ₚ-COOR aus dem ersten Schritt einer Kreuzmetathese unterwirft und deren Produkt hydriert.

8. Verfahren nach Anspruch 1 zur Synthese der Disäure der Formel ROOC-(CH₂)₁₂-COOR aus Vaccensäure mit einem ersten Schritt einer Ethenolyse dieser Säure zu der ungesättigten Säure CH₂=CH- (CH₂)₉-COOR und einem anschließenden zweiten Schritt einer Kreuzmetathese mit der Säure CH₃-CH=CH-CH₂-COOR und abschließender Hydrierung des Produkts des zweiten Schritts.

9. Verfahren nach Anspruch 1 zur Synthese der Disäure der Formel ROOC-(R₂)₈-COOR aus 5-Lauroleinsäure oder 5-Physeterinsäure mit einem ersten Schritt einer Ethenolyse dieser Säure zur Herstellung der Säure der Formel CH₂=CH-(CH₂)₃-COOR, anschließender Homometathese und abschließender Hydrierung.

## Claims

1. Process for the synthesis of diacids of general formula ROOC-(CH₂)ₓ-COOR, in which x represents an integer between 5 and 24 and R is H, starting from long-chain natural monounsaturated fatty acids comprising at least 10 adjacent carbon atoms per molecule, of formula CH₃-(CH₂)ₙ-CHR₁-CH₂-CH=CH-(CH₂)ₚ-COOR, in which R represents H, R₁ is either H or OH, and n and p, which are identical or different, are indices between 3 and 11, which consists, in a first stage, in converting said natural fatty acid, either by pyrolysis or by ethenolysis, into an ω-monounsaturated fatty acid of general formula CH₂=CH-(CH₂)ₘ-COOR, in which m is equal to p or p+1, depending on the nature of the fatty acid treated and the conversion used, then, in a second stage, in subjecting the product thus obtained to a metathesis reaction, either homometathesis, in order to obtain a compound of formula ROOC-(CH₂)ₘ-CH=CH-(CH₂)ₘ-COOR, or cross-metathesis with a compound of formula R₂OOC-(CH₂)ᵣ-CH=CH-R₃, in which R₂ is H, r is either 0 or 1 or 2 and R₃ is H, CH₃ or COOR₂, in the last case forming a noncyclic molecule, in order to obtain an unsaturated compound of formula ROOC-(CH₂)ₘ-CH=CH-(CH₂)ᵣ-COOR₂, and then, in a third stage, in finally converting, by hydrogenation of the double bond, the unsaturated compound to give a saturated compound.

2. Process according to Claim 1, **characterized in that** the natural monounsaturated acid of general formula CH₃-(CH₂)ₙ-CHOH-CH₂-CH=CH-(CH₂)ₚ-COOR is subjected to a pyrolysis reaction.

3. Process according to Claim 2, **characterized in that** the acid of formula CH₂=CH-(CH₂)ₚ₊₁-COOR resulting from the first stage is subjected to a homometathesis, the product of which, ROOC-(CH₂)ₚ₊₁-CH=CH-(CH₂)ₚ₊₁-COOR, is hydrogenated.

4. Process according to Claim 2, **characterized in that** the acid of formula CH₂=CH-(CH₂)ₚ₊₁-COOR resulting from the first stage is subjected to a cross-metathesis, the product of which obtained is hydrogenated.

5. Process according to Claim 1, **characterized in that** the natural monounsaturated fatty acid of general formula CH₃-(CH₂)ₙ-CHOH-CH₂-CH=CH-(CH₂)ₚ-COOR is subjected to an ethenolysis reaction.

6. Process according to Claim 5, **characterized in that** the acid of formula CH₂=CH-(CH₂)ₚ-COOR resulting from the first stage is subjected to a homometathesis, the product of which, ROOC-(CH₂)ₚ-CH=CH-(CH₂)ₚ-COOR, is hydrogenated.

7. Process according to Claim 5, **characterized in that** the acid of formula CH₂=CH-(CH₂)ₚ-COOR resulting from the first stage is subjected to a cross-metathesis, the product of which obtained is hydrogenated.

8. Process according to Claim 1 for the synthesis of the diacid of formula ROOC-(CH₂)₁₂-COOR starting from vaccenic acid with, in the first stage, an ethenolysis of this acid to give the unsaturated acid CH₂=CH-(CH₂)₉-COOR, followed, in the second stage, by a cross-metathesis with the acid CH₃-CH=CH-CH₂-COOR and, finally, by a hydrogenation of the product from the second stage.

9. Process according to Claim 1 for the synthesis of the diacid of formula ROOC-(CH₂)₈-COOR starting from 5-lauroleic or 5-physeteric acid with, in the first stage, an ethenolysis of said acid to produce the acid of formula CH₂=CH-(CH₂)₃-COOR, followed by a homometathesis finished off by the hydrogenation.
